# EUROPEAN PATENT SPECIFICATION

(11) **EP 2 678 446 B1**
(45) Date of publication and mention of the grant of the patent: **28.06.2017**
(21) Application number: 12749229.6
(22) Date of filing: 24.02.2012
(51) Int. Cl.: C12Q 1/68, G01N 33/574

(54) **PRESENCE OF ERG GENE REARRANGEMENTS AND PROTEIN OVER-EXPRESSION IN LOW GRADE PIN (LG-PIN) IN PROSTATE BIOPSIES**
ANWESENHEIT VON ERG-GENNEUANORDNUNGEN UND PROTEINÜBEREXPRESSIONEN BEI GERINGGRADIGER PIN (LG-PIN) IN PROSTATA-BIOPSIEN
PRÉSENCE DE RÉARRANGEMENTS GÉNIQUES DE ERG ET SUREXPRESSION PROTÉIQUE DANS DES NÉOPLASIES INTRAÉPITHÉLIALES PROSTATIQUES (PIN) DE FAIBLE STADE (LG-PIN) DANS DES BIOPSIES DE PROSTATE

(30) Priority: 24.02.2011 US 201161446300 P
(43) Date of publication of application: 01.01.2014
(73) Proprietor: Ventana Medical Systems, Inc., Tucson, Arizona 85755 (US)
(72) Inventor: PESTANO, Gary, Lafayette, CO 80026 (US); NAGLE, Ray, Tucson, AZ 85719 (US); SATHYANARAYANA, Ubaradka, Tucson, AZ 85755 (US); NAGY, Alexandra, Dea, Oro Valley, AZ 85755 (US); CORTEZ, Connie, Tracy, CA 95377 (US); GARSHA, Karl, Sahuarita, AZ 85629 (US); DITTAMORE, Ryan, San Diego, CA 92131 (US)
(74) Representative: Burger, Alexander
(86) International application number: PCT/US2012/026551
(87) International publication number: WO 2012/116294

(56) References cited:
- US-A- 6 054 320
- US-A1- 2008 254 481
- US-A1- 2009 208 937
- ZHANG SHENGLE ET AL: "Detection of TMPRSS2 gene deletions and translocations in carcinoma, intraepithelial neoplasia, and normal epithelium of the prostate by direct fluorescence in situ hybridization", DIAGNOSTIC MOLECULAR PATHOLOGY : THE AMERICAN JOURNAL OF SURGICAL PATHOLOGY, PART B, LIPPINCOTT WILLIAMS & WILKINS, US, vol. 19, no. 3, September 2010 (2010-09), pages 151-156, XP009184570, ISSN: 1533-4066
- K PARK: "Antibody-based detection of ERG rearrangement-positive prostate cancer.", NEOPLASIA, vol. 12, no. 7, July 2010 (2010-07), pages 590-598, XP055050757, DOI: 10.1593/neo.10726
- NAGY D ET AL: "Concordance of ERG gene rearrangements and ERG protein expression in PIN lesions in prostate needle biopsies", EUROPEAN UROLOGY SUPPLEMENTS, February 2012 (2012-02), pages e937-e937a, XP055193175,
- JONIAU ET AL.: 'Prostatic intraepithelial neoplasia (PIN): importance and clinical management.' EUROPEAN UROLOGY, [Online] vol. 48, 2005, pages 379 - 385, XP027606505 Retrieved from the Internet: <URL:http://www.europeanurology.com/article /S0302-2838(05)00149-1/pdf/Prostatic+Intrae pithelial+Neoplasia+(PIN)%3A+Importance+and +Clinical+Management> [retrieved on 2012-05-11]

## Description

### FIELD OF THE INVENTION

The present disclosure relates to methods for the early stage detection of prostate cancer cells in biological samples which have previously been detectable as a cancer pathology. More specifically, the disclosure relates to a combined anti-ERG immunohisotchemistry assay coupled with an ERG FISH assay which leads to identification of Low Grade PIN that is more likely to be associated with and therefore predictive of cancer.

### BACKGROUND OF THE INVENTION

Early detection of cancer is the key feature in treating cancer patients. In men, prostate cancer is the most prevalent form of cancer for all races. While each year over 300,000 men are diagnosed with prostate cancer in the U.S. alone, the currently available tests are notoriously inaccurate and subjective. As a result many incidences of prostate cancer are undiagnosed until the disease has progressed to late stages, including metastases. Both the incidence of prostate cancer and its associated mortality have been increasing over the past ten years. It is estimated that about 50-65% of the prostate cancer is localized, 9-17% has spread to an area near the prostate and 20-25% has metastasized to other parts of the body.

The screening for prostate cancer is primarily by PSA (a blood test for Prostate Specific Antigen) and DRE (Digital Rectal Exam) testing. Confirmation of cancer is made by examination of tissue samples derived from needle biopsies. These methodologies cannot differentiate between benign disease and cancer. The failure to differentiate can result, for example, in exposure of patients with benign disease to treatments that are unnecessary and have side effects (e.g., impotence and incontinence). Moreover, it is estimated that PSA testing misses 20%-30% of all individuals with cancer. There is a clear need for diagnostics with better sensitivity and specificity.

Zhang et al. (Diagn Mol Pathol, 2010; 19: 151-156) describe a TMPRSS2 dual-color break-apart assay using directly labeled fluorescence *in situ* hybridization (FISH) probes to determine the frequency and specificity of TMPRSS2-associated genetic alterations in formalin-fixed paraffin-embedded samples of prostate carcinoma, PIN, and normal prostate tissue (epithelium) from radical prostatectomy specimens.

Park et al. (Neoplasia, 2010; 12(7): 590-598) correlated ERG protein expression with the presence of ERG gene rearrangements in prostate cancer tissues using a combined immunohistochemistry (IHC) and FISH analysis.

With the increase in incidence of prostate cancer and the high rate at which this disorder mestastasized, there is a critical need for detection of this disease at the very earliest stages, and for therapies that can target potentially metastatic prostate cancer cells. Ideally, these types of treatments would have application to both early stage, confined prostate cancer as well as in the treatment of metastatic disease.

One area that would greatly benefit male health is if there were a specific detection method that can be used to detect low grade prostate intraepithelial neoplasia (LG-PIN). LG-PIN is a premalignant proliferation in prostate cells, most commonly found in the peripheral zone. PIN will be identified in up to 16% of men who have had transrectal ultrasound guided prostate biopsies. In PIN the cellular arrangement shows preservation of duct and gland architecture with progressive disruption of the basal cell layer with increasing grades of PIN while invasion of the stroma is lacking. Other histologic or biologic changes that have been reported include: loss of neuroendocrine and secretory differentiation, nuclear and nucleolar abnormalities, neovascularity, increased proliferative potential and genetic instability with variation of DNA content. With increasing degrees of PIN an increasing degree of nuclear aberration is seen along with increasing basal cell disruption. PIN has an intact or fragmented basal cell layer, whereas cancer (PC) lacks a basal cell layer. Basal cell specific immunostaining for cytokeratin is present in PIN but is absent in areas of PC.

PIN is graded from the lowest grade (Grade I) with the least changes to the highest grade (Grade III) with the most severe changes. Most medical papers categorize PIN as either high grade or low grade. High grade PIN and Grade III PIN are used synonymously. PC is associated more with high grade PIN than low grade. PIN appears to predate the appearance of PC by more than 5 years. There is a significant correlation between a finding of high grade PIN and diagnosis of prostate cancer in subsequent biopsies. HG-PIN correlated to a such a subsequent diagnosis in 33-100% of cases in various studies. In one study, PIN was detected in a total of 66 men: 21 with low grade PIN and 45 with high grade PIN. Repeat biopsies revealed PC in 5/21 or 24% of the low grade group and in 26/45 or 58% of the high grade group.

Thus, methods of detection of low grade PIN, which is earlier still in the disease progression cycle as compared to high grade PIN, that could lead to development of cancer could be used for the early stage detection and prognosis of prostate cancer and lead to better treatment regimens for prostate cancer.

Joniau et al. (European Urology, 2005; 48: 379-385) provide a review of approaches and criteria for the early detection of prostate cancer. In particular, the document revolves around prostatic intraepithelial neoplasia (PIN). The authors come to the conclusion that PIN is considered to be the most likely precursor of prostate cancer, and the finding of isolated high grade PIN in prostate biopsies should prompt the clinician to take actions, such as repeat biopsies or chemoprevention trials. Further, it is mentioned that LG PIN cannot be ignored, and this assumption is made on studies wherein 30% of patients had cancer in the LG PIN group.

### BRIEF SUMMARY OF THE INVENTION

The invention relates to a method for early stage diagnosis of prostate cancer in a subject comprising determining the presence of low-grade prostate intraepithelial neoplasia (LG-PIN) in said subject comprising: providing a sample from the subject; detecting the presence or absence in the sample of a gene fusion having a 5' portion from a transcriptional regulatory region of an androgen regulated gene and a 3' portion from an ERG gene by detecting chromosomal rearrangements of genomic DNA using a nucleic acid hybridization technique, and detecting the over-expression of ERG protein using an immunohistochemistry assay wherein the presence in the sample of both the gene fusion and an over expression of ERG is indicative of low-grade PIN in the subject that relates to presence of cancer or is predictive of cancer and wherein said subject has previously been determined as negative for prostate cancer.

In preferred embodiments, the androgen regulated gene may be selected from the group inclusive of TMPRSS2, NDRG1, SLC45A3, and PSA. In other embodiments, the detection of the presence or absence of a gene fusion in the sample comprises detecting chromosomal rearrangements of genomic DNA using a nucleic acid hybridization technique selected from the group consisting of in situ hybridization (ISH), microarray and Southern blot.

In certain embodiments, such detecting comprises detecting chimeric mRNA transcripts having a 5' portion from a transcriptional regulatory region of an androgen regulated gene and a 3' portion from ERG.

In the diagnostic methods of the invention, the in situ hybridization is fluorescence in situ hybridization (FISH) utilizing a probe selected from the group consisting a 5p probe is developed from RP11-95121 and CTD-2506J13 and is located on chromosome 21q22.3 and a ERG 3p probe developed from RP11-476D17 and RP11-24A11 is located on chromosome 21q22.3.

In exemplary methods the detection of overexpression of the ERG protein comprises detecting an amino-terminally truncated ERG protein resulting from a fusion of a transcriptional regulatory region of an androgen regulated gene to an ERG gene. In still other embodiments, the overexpression detection comprises detecting a chimeric protein having an amino-terminal portion encoded by a transcriptional regulatory region of an androgen regulated gene and a carboxy-terminal portion from ERG gene.

The inventive diagnostic methods may be performed on any sample that would be screened for determining prostate cancer. For example, the sample may be selected from the group consisting of tissue, blood, urine, semen, prostatic secretions and prostate cells.

In specific embodiments, the gene fusion is fusion of an ARG (including but not limited to the TMPRSS2) gene and the ERG gene, and wherein the method further comprises the step of characterizing the prostate cells based on the presence or absence of a genomic deletion in the gene fusion. TMPRSS2-ERG rearrangements are known to those of skill in the art. In exemplary embodiments, such a gene rearrangement is a deletion of genomic DNA between the TMPRSS2 gene and the ERG gene on chromosome 21. More particularly, the deletion includes the deletion of exon 1 of the ERG gene or includes the deletion of exon 3 of the TMPRSS2 gene. For example, the deletion comprises a deletion wherein between 2.8 and 2.85 megabases of genomic DNA are deleted.

The deletion may be detected using a FISH assay using at least one fluorescently labeled probe selected from the group consisting of RP11-95121, CTD-2506J13, RP11-476D17 and RP11-24A11. In exemplary embodiments, the deletion is indicative of metastatic prostate cancer in the subject.

The inventive diagnostic methods may further comprise staining said prostate cells using a hematoxylin and eosin (H&E) stain to determine the presence of atypical luminal cells with enlarged nuclear size without visible nucleoli as compared to normal adjacent cells. Alternatively, or in addition, the diagnostic methods may further comprise determining the presence of prostate specific antigen (PSA) in said prostate cells.

As noted, the inventive diagnostic methods advantageously provide an early identification of prostate cancer where other methods have indicated a sample negative for prostate cancer. For example, the disclosed methods are conducted on subjects that have initially tested negative for prostate cancer as determined by needle biopsy and/or as determined by H&E staining.

Also disclosed is an assay for prostate cells for the presence of LG-PIN, comprising: a) obtaining a test sample of prostate cells; b) analyzing the sample of prostate cells to determine the expression of ERG using an immunohistochemistry assay; c) comparing the expression level determined in step b) with the level in a control sample; d) performing a FISH assay to determine presence or absence in the sample of a gene fusion having a 5' portion from a transcriptional regulatory region of an androgen regulated gene and a 3' portion from an ERG gene and e) determining that said prostate cells will develop cancer or are indicative of proximal [or adjacent] cancer cells in said subject if the level of expression of ERG in the prostate cells in said test sample is higher than that for the control sample and there is a presence of gene fusion of a 5' portion from a transcriptional regulatory region of an androgen regulated gene and a 3' portion from the ERG gene as determined in step e).

### BRIEF DESCRIPTION OF SEVERAL VIEWS OF THE DRAWINGS

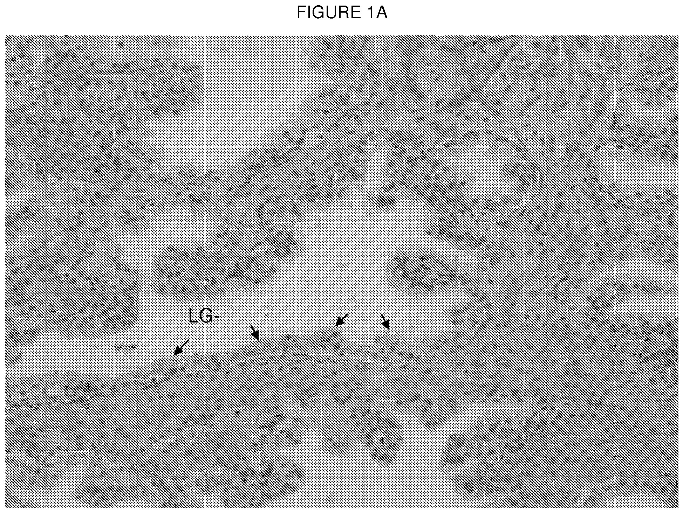

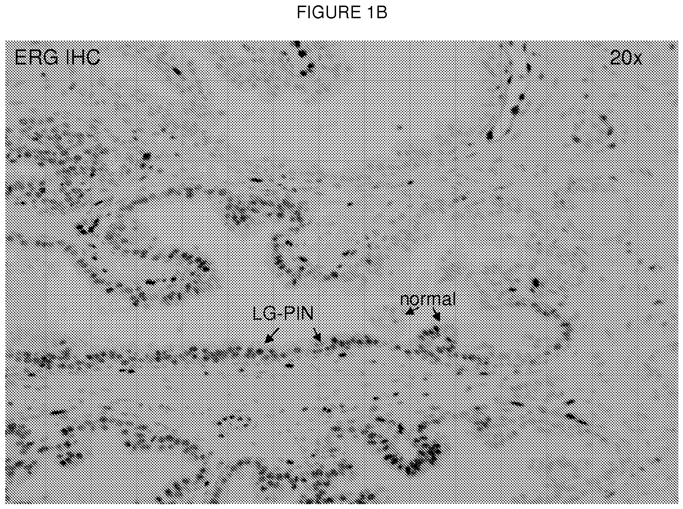

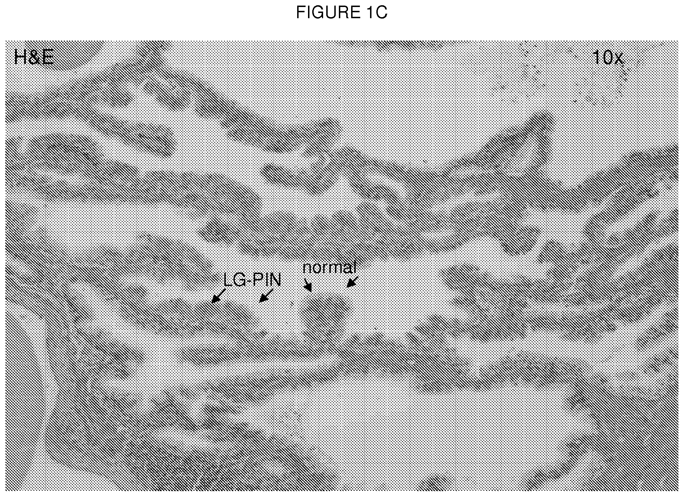

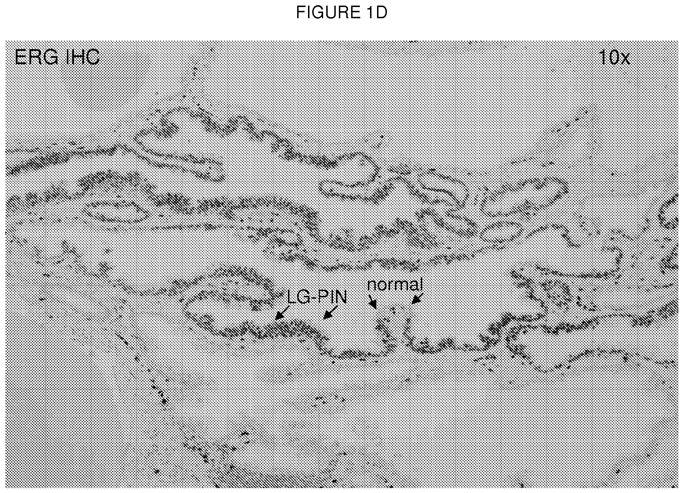

Figure 1: Low-Grade PIN in two cases showing (1A) H&E staining of CASE 1, and (1B) ERG over-expression by IHC in a serial cut slide section of CASE 1; (1C) H&E staining of CASE 2, and (1D) ERG over-expression by IHC in a serial cut slide section of CASE 2. Note: LG-PIN is diagnosed on the H&E by atypical luminal cells with enlarged nuclear size without visible nucleoli as compared to normal adjacent cells.
Figure 2: ERG Rearrangements in prostate break-apart assay.

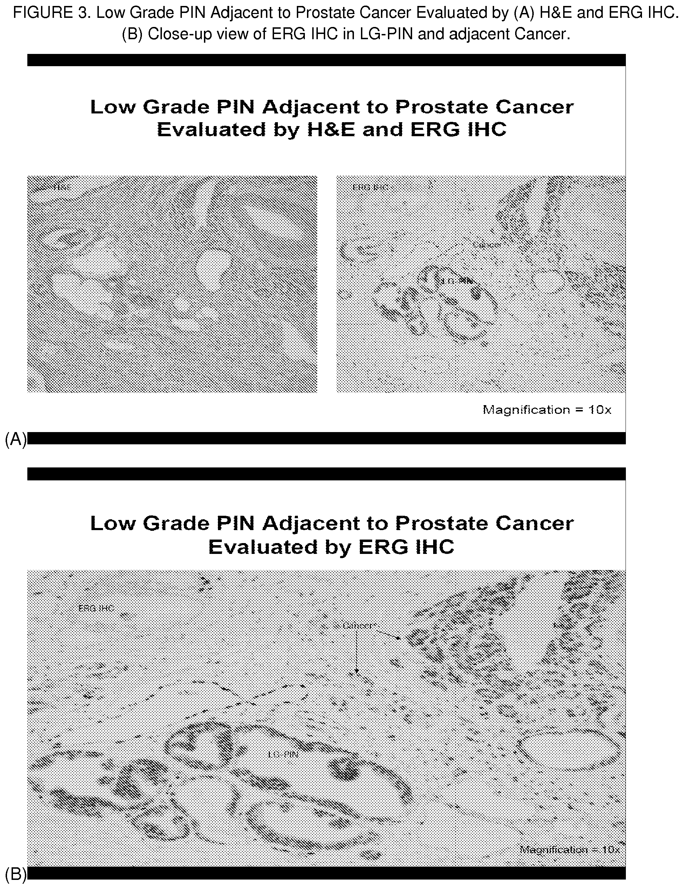

Figure 3 Low Grade PIN Adjacent to Prostate Cancer Evaluated by H&E and ERG IHC.

### DETAILED DESCRIPTION OF THE INVENTION

Currently the pathology community does not report on Low-grade PIN. The diagnosis by H&E staining is not well characterized and is largely subjective, thus there remains unclear whether there exists a direct association between LG-PIN and progression to prostate adenocarcinoma. The current disclosure extends on the finding that ETS gene arrangements are present in prostate cancer. The disclosed methods provide evidence for ERG gene rearrangements and associated ERG protein over-expression in Low Grade PIN. The present disclosure shows there is potential to identify a novel subset of Low Grade PIN that may be more likely to be associated with or progress to prostate carcinoma. These findings may be useful in clinical practice to provide methods of prognosticating a man's risk of prostate cancer that may have been missed by needle biopsy and hence provide a more sensitive and accurate prediction of likelihood of having or developing prostate cancer.

The current practice for evaluating prostate cancer is to not report on Low Grade PIN. Instead, this category of patient is subjected to re-screening using PSA monitoring, repeated DRE and multiple re-biopsies as needed until definitive evidence of cancer is found. The advantage of the present disclosure is it provides an improved method of predicting cancer as compared to biopsies alone thereby reducing the need for unnecessary biopsies and providing an early stage diagnosis of prostate cancer.

The discovery of TMPRSS2:ERG gene rearrangement in prostate cancers and its association with disease progression has both therapeutic and diagnostic implications. Identification of men who are elevated for risk of progression from pre-neoplastic PIN lesions to prostate carcinoma is an unmet medical need which may directly impact the practice of needle biopsy sampling and PSA screening. Previous studies have identified the presence of ERG rearrangements in approximately 15% of high grade PIN. In addition, ERG rearranged HG PIN lesions are highly associated with gene rearrangements in adjacent tumor foci in prostatectomy specimens. In the present disclosure, a diagnostic method focuses on determining the presence of both ERG gene rearrangements and protein over-expression in LG-PIN as correlative of cancer and uses the same to identify men who are at risk of more rapidly developing prostate carcinoma.

For such diagnostic purposes, a prostate tissue sample may be taken from an individual and tested for the presence of the ERG fusions and for the over-expression of ERG. The tissue sample can exclude the single cell layer of cell lining the glandular epithelium, or include cells both within and outside the layer. By way of nonlimiting examples, the sample may be tissue (e.g., a prostate biopsy sample or a tissue sample obtained by prostatectomy), blood, urine, semen, prostatic secretions or a fraction thereof (e.g., plasma, serum, urine supernatant, urine cell pellet or prostate cells). A urine sample is preferably collected immediately following an attentive digital rectal examination (DRE), which causes prostate cells from the prostate gland to shed into the urinary tract. In certain embodiments, the prostate tissue sample may be the result of a biopsy procedure. The prostate tissue sample may be a plurality of contiguous cells and/or one or more separate cells from the prostate.

The disclosed diagnostic/prognostic testing methods also may be combined with PSA testing and/or DRE. Tests for prostate specific antigen (PSA) are well known in the art. As noted above, screening for PSA to detect prostate cancer is estimated to miss (i.e., false negatives) a significant proportion of the individuals tested who actually have prostate cancer. An advantage of the present invention is that the method of screening for prostate cancer may detect prostate cancer in an individual having the cancer but who tests negative in a test for PSA. The present method may be used alone or in combination with a test for PSA.

For therapeutic purposes, it is desirable to be able to determine whether a patient diagnosed or presenting with prostate dysfunction has a benign prostate disease or prostate cancer. Benign prostate disease includes benign prostatic hyperplasia (BPH). Advantageously, in the disclosed methods the detection of LG-PIN correlates with prostate cancer thereby allowing the skilled person to distinguish from benign prostate disease in a patient diagnosed or presenting with prostate dysfunction. The presence of LG-PIN as determined by the combined ERG IHC-FISH assay described herein is associated with prostate cancer and conversely, the absence of LG-PIN is indicative of benign prostate disease. A patient diagnosed or presenting with prostate dysfunction may have been tested in a test for PSA, and may have tested negative. By combining the PSA testing with the ERG IHC-FISH assay described herein it is possible to provide a sensitive and early stage detection of prostate cancer.

Recurrent gene fusions indicative of prostate cancer have now been characterized for a number of years. The gene fusions are the result of a chromosomal rearrangement of an androgen regulated gene (ARG) and an ETS family member gene, including for example the ERG proto-oncogene. Despite their recurrence, the junction where the ARG fuses to the ETS family member gene varies. The gene fusions typically comprise a 5' portion from a transcriptional regulatory region of an ARG and a 3' portion from an ETS family member gene. The recurrent gene fusions have use as diagnostic and/or prognostic markers and clinical targets for prostate cancer.

The ARGs are regulated by androgenic hormones and are of critical importance for the normal physiological function of the human prostate gland. They also contribute to the development and progression of prostate carcinoma. Recognized ARGs include, but are not limited to: TMPRSS2; PSA; PSMA; KLK2; SNRK; Seladin-1; and, FKBP51 (Paoloni-Giacobino et al., Genomics 44: 309 (1997); Velasco et al., Endocrinology 145(8): 3913 (2004)). TMPRSS2 (NM.sub.--005656), in particular, has been demonstrated to be highly expressed in prostate epithelium relative to other normal human tissues (Lin et al., Cancer Research 59: 4180 (1999)). The TMPRSS2 gene is located on chromosome 21. This gene is located at 41,750,797-41,801,948 bp from the pter (51,151 total bp; minus strand orientation).

The transcriptional regulatory region of an ARG may contain coding or non-coding regions of the ARG, including the promoter region.

The ETS family of transcription factors regulate the intra-cellular signaling pathways controlling gene expression. As downstream effectors, they activate or repress specific target genes. As upstream effectors, they are responsible for the spatial and temporal expression of numerous growth factor receptors. Almost 30 members of this family have been identified and implicated in a wide range of physiological and pathological processes. These include, but are not limited to: ERG; ETV1 (ER81); FLI1; ETS1; ETS2; ELK1; ETV6 (TEL1); ETV7 (TEL2); GABP.alpha.; ELF1; ETV4 (E1AF; PEA3); ETV5 (ERM); ERF; PEA3/E1AF; PU.1; ESE1/ESX; SAP1 (ELK4); ETV3 (METS); EWS/FLI1; ESE1; ESE2 (ELF5); ESE3; PDEF; NET (ELK3; SAP2); NERF (ELF2); and FEV.

The fusion of an ARG to an ETS family member gene is detectable as DNA, RNA or protein. Initially, the gene fusion is detectable as a chromosomal rearrangement of genomic DNA having a 5' portion from a transcriptional regulatory region of the ARG and a 3' portion from the ETS family member gene. Once transcribed, the gene fusion is detectable as a chimeric mRNA having a 5' portion from the transcriptional regulatory region of the ARG and a 3' portion from the ETS family member gene. Once translated, the gene fusion is detectable as an amino-terminally truncated ETS family member protein resulting from the fusion of the transcriptional regulatory region of the ARG to the ETS family member gene; a chimeric protein having an amino-terminal portion from the transcriptional regulatory region of the ARG and a carboxy-terminal portion from the ETS family member gene; or, an upregulated, but otherwise indistinguishable, native ETS family member protein. The truncated ETS family member protein and chimeric protein may differ from their respective native proteins in amino acid sequence, post-translational processing and/or secondary, tertiary or quaternary structure. Such differences, if present, can be used to identify the presence of the gene fusion. In a preferred embodiment, FISH is used to detect the presence of the gene fusion.

### ERG Immunohistochemistry Assay

Evaluation of ERG Protein Expression by IHC may be accomplished using manual and automated methods. The following methods are referenced in the seminal publication showing the correlation between ERG IHC and FISH (from Neoplasia, Volume 12 Number 7 July 2010 pp. 590-598). This reference is only cited as an example of conducting ERG IHC. Alternative automated platforms, and manual methods may yield similar results but have not yet been demonstrated in the literature.

Immunohistochemical (IHC) analyses on paraffin-embedded formalin-fixed tumor tissue sections were carried out using the automated DiscoveryXT staining platform from Ventana Medical Systems. The primary rabbit monoclonal antibody was obtained froma commercial source. Antigen recovery was conducted using heat retrieval and CC1 standard, a high pH Tris/borate/EDTA buffer (VMSI, catalog no. 950-124). Slides were incubated with 1:100 of the ERG primary antibody for 1 hour at room temperature. Primary antibody was detected using the ChromoMap DAB detection kit (VMSI, catalog no.760-159) and UltraMap anti-Rb HRP (VMSI, catalog no. 760-4315). The anti-Rb HRP secondary antibody was applied for 16 minutes at room temperature. Slides were counterstained with Hematoxylin II (VMSI, catalog no. 790-2208) for 8 minutes followed by Bluing Reagent (VMSI, catalog no. 760-2037) for 4 minutes at 37°C.

### FISH Assay for detection of ERG fusion

Assessment of Gene Rearrangement Status Using Two-color Interphase FISH may be conducted using manual and automated methods. The following methods are referenced in the seminal publication showing the correlation between ERG IHC and FISH (from Neoplasia, Volume 12 Number 7 July 2010 pp. 590-598). This reference is only cited as an example of conducting ERG FISH. Automated platforms, and manual methods may yield similar results. Four-micrometer-thick TMA sections were used for interphase FISH analysis. Rearrangement status was determined using a dual-color break-apart interphase FISH assay as described previously [Tomlins SA, Rhodes DR, Perner S, Dhanasekaran SM, Mehra R, Sun XW, Varambally S, Cao X, Tchinda J, Kuefer R, et al. (2005). Recurrent fusion of TMPRSS2 and ETS transcription factor genes in prostate cancer. Science 310(5748), 644-648. and Perner S, Demichelis F, Beroukhim R, Schmidt FH, Mosquera JM, Setlur S, Tchinda J, Tomlins SA, Hofer MD, Pienta KG, et al. (2006). TMPRSS2: ERG fusion-associated deletions provide insight into the heterogeneity of prostate cancer. Cancer Res 66(17), 8337-8341].

In addition, an automated FISH assay using ERG 3p and 5p FISH probes and Quantum Dots 565 and 655yields similarly high correlation with the ERG IHC assay for the detection of gene rearrangements A summary of the methods employing Quantum Dot detection is reported in the chart reproduced below:

**3.5' and 3' ERG Break-apart Automated Quantum Dot FISH Method.**

| **Platform** | **BenchMark XT** |
|---|---|
| Deparaffinization | Extended |
| Cell Conditioning | Extended/Reaction Buffer |
| Protease digestion | Protease 3, 12 min |
| Denaturation | 90°C, 12 min |
| Hybridization | 44°C, 8 hours |
| Stringency Wash | 72°C, 8 min (3 cycles) |
| *5p ERG* DIG/ *3p ERG* DNP | 2 drops each |
| Blocking Buffer | 1 drop, 32 min at Room Temp |
| Detection | QD 565 and QD 655, 32 min, RT |
| Counterstain: DAPI | 4 min at Room Temp |
| Coverslip | Cytoseal 60 |

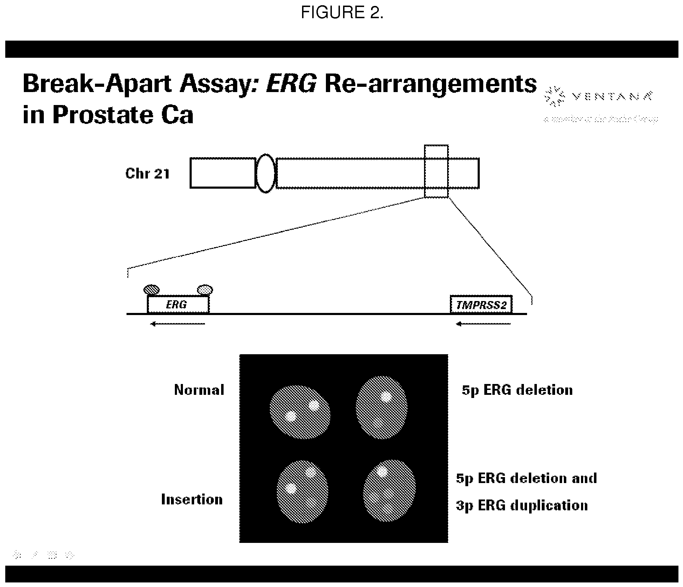

Probes that exhibit no rearrangement, i.e., both alleles are "normal", will remain co-localized, and are visualized as either a pair of yellow signals, or as very closely juxtaposed red/green signals. Rearrangements of ERG will yield an "insertion" of the fluorescent probes as depicted in FIGURE 2. Break-apart events may be visualized as a result of the "insertions" in the 5p region of ERG, and may be accompanied by "deletion" of one of the 5p ERG (green) signals, as well as duplication of the 3p ERG gene.

### Examples

The goal of this study was to identify the extent of ERG rearrangements in PIN lesions of the prostate. The inventors developed an automated staining procedure to detect ERG over-expression by immunohistochemistry (IHC), in combination with the specific gene rearrangements detected by a FISH assay. To this end, probes specific to the ERG gene rearrangements including 3' and 5' ERG were detected with Quantum Dot (LifeTechnologies, OR) bioconjugates. Fixatives commonly used in preservation of needle biopsies as well as fixation times were also evaluated. Specific FISH signals were deconvolved using an interferometer and spectral imaging software.

Specific methods for conducting spectral FISH in formalin-fixed and paraffin-embedded tissues with Quantum Dots are described in the literature. Briefly, spectral image cubes containing high-resolution wavelength intensity information at each pixel were captured using a modified SpectraView (ASI; Applied Spectral Imaging, Israel) acquisition and analysis system. The modified system comprises a computer workstation, fluorescent microscope (Olympus BX61), a light-guide coupled, and stabilized metal halide excitation source (Exfo Exacte, Exfo, Ontario, CA, USA), with spectral output from an interferometer optical head to a Sony ICX285 digital CCD.20-23 The excitation/emission filters for spectral imaging (Semrock, USA) were as follows: 377nm center wavelength with 50nm bandwidth for excitation; dichroic beamsplitter with reflection band below 410nm, and a long pass filter with deep blocking transition at 409 nm. All spectral image cubes were captured with a 40X plan-fluor objective (numerical aperture 0.75) and 1Xc-mount. Data were gathered through a series of 100 ms exposures to build the interferometric image cube for spectral processing. The wavelength range represented by image cubes captured under these conditions covered the visible wavelengths between 410 and 900nm at a 10-nm wavelength sampling resolution. Spectral unmixing to separate signals corresponding to tissue autofluorescence, DAPI, Quantum Dot 565, and Quantum Dot 655 was conducted using the appropriate reference spectra and linear unmixing algorithms implemented in SpectraView spectral data analysis software.

The distinctive narrow Gaussian wavelength distribution and discreet peak locations of the QD emission spectra enabled reliable separation of the individual probe signals. The individual monochrome DAPI and FISH signal intensity layers were colorized and merged to provide overlay images for visualization of relative probe localizations.

The results showed sensitive and specific detection of gene rearrangements with this testing method in the xenograft models VCaP, H660 and LNCap, as well as in samples from prostate needle biopsies, and radical prostatectomies. In a cohort of prostate tissue specimens, the ERG IHC was diagnostic of Low Grade PIN that was missed in the initial examination by H&E staining (n = 4/10 or 40% of LG-PIN specimens; TABLE 1). Significantly, although the association of LG-PIN with cancer was relatively infrequent (only 10%, or 6 of 60 specimens were observed to have LG-PIN proximal to cancer); the frequency of LG-PIN proximal to cancer when ERG is rearranged is 75% (that is 3 of 4 LG-PIN specimens that were ERG positive were proximal to cancer, only 1 LG-PIN/ERG positive specimen was not immediately proximal to cancer (TABLE 1).

Similarly for HG-PIN, although the frequency of association with cancer was relatively low at 25% (15 of 60); when ERG is expressed in HG-PIN the proximity of cancer is 100% (4 of 4 cases); that is , all cases of ERG positive HG-PIN were associated with proximal cancer (TABLE 1).

**TABLE 1. Occurrence of ERG Rearrangements in PIN Samples, including LG-PIN and HG-PIN in a Prostate Specimen Cohort (A) All data for PIN, Cancer and Non-Cancer Specimens, and (B) Summary for LG-PIN and (C) HG-PIN Co-occurrence with Cancer. (A)**

| | **Number of Cases** | **ERG Negative** | **ERG Positive** |
|---|---|---|---|
| Total Cases* | 89 | 68 | 21 |
| Total Cancer | 60 | 40 | 20 |
| Total Non-Cancer Cases (includes PIN) | 29 | | |
| | | | |
| Total co-occurrence of CA and PIN: | 27 | 19 | 8 |
| **Total cases with LG PIN** | **10** | **6** | **4** |
| Total cases with HG PIN | 17 | 13 | 4 |
| **Co-occurrence of Cancer and LG PIN** | **6** | **3** | **3** |
| Co-occurrence of Cancer and HG PIN | 15 | 11 | 4 |
| | | | |
| Total Non-Cancer Cases (includes PIN) | **29** | | |
| **LGPIN with No Cancer** | **4** | **3** | **1** |
| HGPIN with No Cancer | 2 | 2 | 0 |

| | | | |
|---|---|---|---|
| *one case had HG-PIN, LG-PIN and Cancer | | | |

**(B) LG-PIN Co-occurrence with Cancer**

| | **Percentage** | **n** |
|---|---|---|
| LG-PIN Frequency in Cohort | 11.24% | n = 10 of 89 |
| LG-PIN Associated with Cancer | 10.00% | n = 6 of 60 |
| LG-PIN Positive for ERG | 40.00% | n = 4 of 10 |
| **ERG Positive LG-PIN co-occurring with cancer** | **75.00%** | **n = 3 of 4** |
| ERG Positive LG-PIN without co-occurrence of cancer | 25.00% | n = 1 of 4 |

**(C) HG-PIN Co-occurrence with Cancer**

| | **Percentage** | **n** |
|---|---|---|
| HG-PIN Frequency in Cohort | 20.22% | n = 18 of 89 |
| HG-PIN Associated with Cancer | 25.00% | n = 15 of 60 |
| HG-PIN Positive for ERG | 23.53% | n = 4 of 17 |
| **ERG Positive HG-PIN co-occurring with cancer** | **100.00%** | **n = 4 of 4** |
| ERG Positive HG-PIN without co-occurrence of cancer | 0.00% | n = 0 of 4 |

Overexpression of ERG was not significantly associated with any specific type of gene rearrangement, including insertions or deletions in the 5' region of the ERG gene. The ERG FISH test assessed rearrangements include: no rearrangement (normal), translocation through insertion, and translocation through deletion.

Table 2 below shows ERG overexpression and gene rearrangement in a selected cohort of prostate tissue samples.

**TABLE 2:**

| **Diagnosis** | **Case ID** | **H&E** | **ERG IHC** | **ERG FISH** |
|---|---|---|---|---|
| **Normal** | PR043 | Benign | - | - |
| | PR046 | Benign | - | - |
| | PR057 | Benign | - | - |
| | PR059 | Benign | - | - |
| | PR068 | Benign | - | - |
| **Pre-neoplastic** | PR042 | LG PIN | + | + |
| | PR052 | LG PIN | - | - |
| | EU003 | HG PIN | - | - |
| **Tumor/ No Rearrangement** | PR003 | Gleason 3+3 | - | - |
| | PR004 | Gleason 3+3 | - | - |
| | PR013 | Gleason 3+3 | - | - |
| | PR024 | Gleason 3+3 | - | - |
| | PR037 | Gleason 3+3 | - | - |
| | EU018 | Gleason 4+3 | - | - |
| | *PR036 | Gleason 3+3 | + | - |
| | PR005 | Gleason 3+3 | + | + |
| | PR010 | Gleason 3+3 | + | + |
| | PR014 | Gleason 3+4 | + | + |
| | PR025 | Gleason 3+3 | + | + |
| | PR028 | Gleason 3+4 | + | + |
| | PR030 | Gleason 3+3 | + | + |
| | PR032 | Gleason 5+5 | ++ | + |
| **Tumor/** | PR035 | Gleason 4+3 | + | + |
| **Rearrangement** | PR038 | Gleason 3+3 | + | + |
| | EU004 | Gleason 3+4 | + | + |
| | EU007 | Gleason 4+3 | + | + |
| | EU025 | Gleason 4+3 | + | + |
| | EU026 | Gleason 4+4 | + | + |
| | EU027 | Gleason 3+4 | + | + |
| | EU029 | Gleason 4+3 | + | + |

| | | | | |
|---|---|---|---|---|
| *Example of Discordant ERG over-expression and Gene Rearrangement | | | | |

In brief, the data indicate that an anti-ERG immunohistochemistry assay coupled with an ERG Quantum Dot FISH provides a sensitive and specific reflex test format that may have utility - subsequent to initial H&E staining - in detecting clinically relevant biomarkers in PIN lesions. Identification of gene rearrangements and ERG protein overexpression in Low Grade PIN may help define a new class of more aggressive prostate disease. This test format may help identify men who should be considered for more aggressive monitoring, for example through re-biopsy, active PSA screening, DRE and MRI examinations. Studies indicate that this test strategy provides prognostic value in assessing prostate cancer progression associated with the incidence of LG-PIN in selected prostate cancer and biopsy cohorts.

## Claims

1. A method for early stage diagnosis of prostate cancer in a subject comprising determining the presence of low-grade prostate intraepithelial neoplasia (LG-PIN) in said subject comprising:
(a) providing a sample from the subject, the subject having previously been deemed negative for prostate cancer;
(b) detecting the presence or absence in the sample of a gene fusion having a 5' portion from a transcriptional regulatory region of an androgen regulated gene and a 3' portion from an ERG gene by detecting chromosomal rearrangements of genomic DNA using a nucleic acid hybridization technique, and
(c) detecting the expression level of ERG protein using an immunohistochemistry assay
wherein the presence in the sample of both the gene fusion as detected in step (b) and an over expression of ERG as detected in step (c) is indicative of low-grade PIN in the subject.

2. The method of claim 1, wherein the sample is a sample of prostate cells, and wherein in step (b) a fluorescence in situ hybridization (FISH) assay is performed,
the method further comprising:
(d) comparing the expression level of ERG protein as detected in step (c) with the expression level of ERG protein in a control sample, and
(e) determining that said prostate cells will become cancerous or are indicative of cancer cells adjacent to the sample locus in said subject if the level of expression of ERG in the prostate cells in said test sample is higher than that for the control sample and there is a presence of gene fusion of a 5' portion from a transcriptional regulatory region of an androgen regulated gene and a 3' portion from the ERG gene as determined in step (b).

3. The method of claim 1 or 2, wherein the androgen regulated gene may be selected from the group inclusive of TMPRSS2, NDRG1, SLC45A3, and PSA.

4. The method of claim 1, wherein step (b) comprises detecting chromosomal rearrangements of genomic DNA using a nucleic acid hybridization technique selected from the group consisting of in situ hybridization, microarray and Southern blot, or wherein step (b) comprises detecting chimeric mRNA transcripts having a 5' portion from a transcriptional regulatory region of an androgen regulated gene and a 3' portion from ERG.

5. The method of claim 2 or 4, wherein said in situ hybridization is fluorescence in situ hybridization (FISH) utilizing a probe selected from the group consisting a 5p probe developed from RP11-95I21 and CTD-2506J13 and is located on chromosome 21q22.3 and a ERG 3p probe developed from RP11-476D17 and RP11-24A11 and is located on chromosome 21q22.3.

6. The method of claim 1, wherein step (c) comprises detecting an amino-terminally truncated ERG protein resulting from a fusion of a transcriptional regulatory region of an androgen regulated gene to an ERG gene, or wherein step (c) comprises detecting a chimeric protein having an amino-terminal portion encoded by a transcriptional regulatory region of an androgen regulated gene and a carboxy-terminal portion from ERG gene.

7. The method of claim 1, wherein the gene fusion is a fusion of an androgen regulated gene and the ERG gene, and wherein the method further comprises the step of characterizing the prostate cells based on the presence or absence of a genomic deletion in the gene fusion.

8. The method of claim 7, wherein said genomic deletion is a deletion of genomic DNA between the TMPRSS2 gene and the ERG gene on chromosome 21.

9. The method of claim 8, wherein said deletion includes the deletion of exon 1 of the ERG gene, or wherein said deletion includes the deletion of exon 3 of the TMPRSS2 gene.

10. The method of claim 8, wherein said deletion is a deletion of between 2.8 and 2.85 megabases of genomic DNA.

11. The method of claim 10, wherein said deletion is detected using a FISH assay using at least one fluorescently labeled probe selected from the group consisting of RP11-95I21, CTD-2506J13, RP11-476D17 and RP11-24A11.

12. The method of claim 10, wherein the presence of the deletion is indicative of metastatic prostate cancer in the subject.

13. The method of claim 1, further comprising staining prostate cells of said sample using a hematoxylin and eosin stain to determine the presence of atypical luminal cells with enlarged nuclear size without visible nucleoli as compared to normal adjacent cells.

14. The method of claim 1, further comprising determining the presence of prostate specific antigen (PSA) in prostate cells of said sample.

15. The method of claim 1, wherein said subject is negative for prostate cancer as determined by needle biopsy.

## Patentansprüche

1. Verfahren zur Diagnose im Frühstadium von Prostatakrebs in einem Individuum, umfassend das Bestimmen der Anwesenheit einer geringgradigen prostatischen intraepithelialen Neoplasie (LG-PIN) in dem Individuum, umfassend:
(a) Bereitstellen einer Probe von dem Individuum, wobei das Individuum zuvor als negativ für Prostatakrebs erachtet wurde;
(b) Nachweisen der Anwesenheit oder Abwesenheit einer Genfusion in der Probe mit einem 5'-Abschnitt aus einer transkriptionsregulierenden Region eines Androgen-regulierten Gens und einem 3'-Abschnitt aus einem ERG-Gen durch Nachweisen chromosomaler Neuanordnungen von genomischer DNA unter Verwendung einer Nukleinsäure-Hybridisierungstechnik, und
(c) Nachweisen des Expressionsniveaus von ERG-Protein unter Verwendung eines Immunhistochemie-Assays,
wobei die Anwesenheit in der Probe sowohl der Genfusion, wie in Schritt (b) nachgewiesen, als auch einer Überexpression von ERG, wie in Schritt (c) nachgewiesen, auf eine geringgradige PIN in dem Individuum hindeutet.

2. Verfahren nach Anspruch 1, wobei die Probe eine Probe von Prostatazellen ist und wobei in Schritt (b) ein Fluoreszenz-in-situ-Hybridisierungs(FISH)-Assay durchgeführt wird,
wobei das Verfahren ferner umfasst:
(d) Vergleichen des Expressionsniveaus von ERG-Protein, wie in Schritt (c) nachgewiesen, mit dem Expressionsniveau von ERG-Protein in einer Kontrollprobe, und
(e) Bestimmen, dass die Prostatazellen krebsartig werden oder auf Krebszellen benachbart zu dem Probenort in dem Individuum hindeuten, wenn das Niveau der Expression von ERG in den Prostatazellen in der Testprobe höher ist als jenes für die Kontrollprobe, und es eine Anwesenheit einer Genfusion eines 5'-Abschnitts aus einer transkriptionsregulierenden Region eines Androgen-regulierten Gens und eines 3'-Abschnitts aus dem ERG-Gen, wie in Schritt (b) bestimmt, gibt.

3. Verfahren nach Anspruch 1 oder 2, wobei das Androgen-regulierte Gen aus der Gruppe einschließlich TMPRSS2, NDRG1, SLC45A3 und PSA ausgewählt ist.

4. Verfahren nach Anspruch 1, wobei Schritt (b) das Nachweisen chromosomaler Neuanordnungen von genomischer DNA unter Verwendung einer Nukleinsäure-Hybridisierungstechnik umfasst, ausgewählt aus der Gruppe bestehend aus In-situ-Hybridisierung, Mikroarray und Southern-Blot, oder wobei Schritt (b) das Nachweisen chimärer mRNA-Transkripte mit einem 5'-Abschnitt aus einer transkriptionsregulierenden Region eines Androgen-regulierten Gens und einem 3'-Abschnitt aus ERG umfasst.

5. Verfahren nach Anspruch 2 oder 4, wobei die In-situ-Hybridisierung eine Fluoreszenz-in-situ-Hybridisierung (FISH) unter Verwendung einer Sonde ist, die aus der Gruppe ausgewählt ist, die aus einer 5p-Sonde, die aus RP11-95I21 und CTD-2506J13 entwickelt wurde und sich auf Chromosom 21q22.3 befindet, und einer ERG-3p-Sonde besteht, die aus RP11-476D17 und RP11-24A11 entwickelt wurde und sich auf Chromosom 21q22.3 befindet.

6. Verfahren nach Anspruch 1, wobei Schritt (c) das Nachweisen eines aminoterminal verkürzten ERG-Proteins umfasst, das aus einer Fusion einer transkriptionsregulierenden Region eines Androgen-regulierten Gens mit einem ERG-Gen resultiert, oder wobei Schritt (c) das Nachweisen eines chimären Proteins mit einem aminoterminalen Abschnitt umfasst, der durch eine transkriptionsregulierende Region eines Androgen-regulierten Gens und einen carboxyterminalen Abschnitt aus dem ERG-Gen codiert ist.

7. Verfahren nach Anspruch 1, wobei die Genfusion eine Fusion eines Androgen-regulierten Gens und des ERG-Gens ist, und wobei das Verfahren ferner den Schritt der Charakterisierung der Prostatazellen auf Basis der Anwesenheit oder Abwesenheit einer genomischen Deletion in der Genfusion umfasst.

8. Verfahren nach Anspruch 7, wobei die genomische Deletion eine Deletion genomischer DNA zwischen dem TMPRSS2-Gen und dem ERG-Gen auf Chromosom 21 ist.

9. Verfahren nach Anspruch 8, wobei die Deletion die Deletion von Exon 1 des ERG-Gens umfasst, oder wobei die Deletion die Deletion von Exon 3 des TMPRSS2-Gens umfasst.

10. Verfahren nach Anspruch 8, wobei die Deletion eine Deletion von zwischen 2,8 und 2,85 Megabasen von genomischer DNA ist.

11. Verfahren nach Anspruch 10, wobei die Deletion unter Verwendung eines FISH-Assays mit mindestens einer fluoreszenzmarkierten Sonde, die ausgewählt ist aus der Gruppe bestehend aus RP11-95I21, CTD-2506J13, RP11-476D17 und RP11-24A11, nachgewiesen wird.

12. Verfahren nach Anspruch 10, wobei die Anwesenheit der Deletion auf einen metastasischen Prostatakrebs in dem Individuum hindeutet.

13. Verfahren nach Anspruch 1, ferner umfassend das Färben von Prostatazellen der Probe unter Verwendung eines Hämatoxylin- und Eosin-Farbstoffs, um die Anwesenheit von atypischen luminalen Zellen mit vergrößerter Kerngröße ohne sichtbare Nukleoli im Vergleich zu normalen benachbarten Zellen zu bestimmen.

14. Verfahren nach Anspruch 1, ferner umfassend das Bestimmen der Anwesenheit von Prostataspezifischem Antigen (PSA) in Prostatazellen der Probe.

15. Verfahren nach Anspruch 1, wobei das Individuum für Prostatakrebs negativ ist, wie durch Nadelbiopsie bestimmt.

## Revendications

1. Méthode de diagnostic précoce du cancer de la prostate chez un sujet comprenant la détermination de la présence d'une néoplasie intraépithéliale prostatique de faible grade (LG-PIN) chez ledit sujet comprenant:
(a) l'utilisation d'un échantillon provenant du sujet, le sujet ayant précédemment été exempt du cancer de la prostate ;
(b) la détection de la présence ou de l'absence dans l'échantillon d'une fusion génique ayant une partie 5' d'une région régulatrice de transcription d'un gène régulé par des androgènes et une partie 3' d'un gène ERG par détection de réarrangements chromosomiques d'ADN génomique au moyen d'une technique d'hybridation d'acide nucléique ; et
(c) la détection du niveau d'expression de la protéine ERG par le biais d'un essai immunohistochimique
où la présence dans l'échantillon à la fois de la fusion génique telle que détectée à l'étape (b) et d'une surexpression de ERG telle que détectée à l'étape (c) indique une PIN de faible grade chez le sujet.

2. Méthode selon la revendication 1, dans laquelle l'échantillon est un échantillon de cellules de prostate, et dans laquelle, dans l'étape (b), un essai d'hybridation in situ fluorescent (FISH) est effectué,
la méthode comprenant en outre :
(d) la comparaison du niveau d'expression de la protéine ERG tel que détecté à l'étape (c) avec le niveau d'expression de la protéine ERG dans un échantillon témoin et
(e) la procédure visant à déterminer si lesdites cellules de prostate sont susceptibles de devenir cancéreuses ou indiquent la présence de cellules cancéreuses adjacentes au site de prélèvement dans ledit sujet si le niveau d'expression de ERG dans les cellules de prostate dans ledit échantillon à tester est plus élevé que celui de l'échantillon témoin et est présente une fusion génique d'une partie 5' d'une région régulatrice de transcription d'un gène régulé par des androgènes et d'une portion 3' du gène ERG telle que déterminée à l'étape (b).

3. Méthode selon la revendication 1 ou 2, dans laquelle le gène régulé par des androgènes peut être choisi dans le groupe comprenant TMPRSS2, NDRG1, SLC45A3 et PSA.

4. Méthode selon la revendication 1, dans laquelle l'étape (b) comprend la détection de réarrangements chromosomiques d'ADN génomique par le biais d'une technique d'hybridation d'acide nucléique choisie dans le groupe constitué par l'hybridation in situ, le microréseau et le transfert de Southern, ou dans laquelle l'étape (b) comprend la détection de transcrits d'ARNm chimériques ayant une partie 5' à partir d'une région régulatrice de transcription d'un gène régulé par des androgènes et d'une partie 3' de ERG.

5. Méthode selon la revendication 2 ou 4, dans laquelle ladite hybridation in situ est une hybridation in situ fluorescente (FISH) qui utilise une sonde choisie dans le groupe constitué par une sonde 5p développée à partir de RP11-95I21 et CTD-2506J13 et se trouvant sur le chromosome 21q22.3 et une sonde ERG 3p développée à partir de RP11-476D17 et RP11-24A11 et se trouvant sur le chromosome 21q22.3.

6. Méthode selon la revendication 1, dans laquelle l'étape (c) comprend la détection d'une protéine ERG tronquée au niveau de son extrémité amino résultant d'une fusion d'une région régulatrice de transcription d'un gène régulé par des androgènes à un gène ERG, ou dans laquelle l'étape (c) comprend la détection d'une protéine chimère ayant une partie à terminaison amino codée par une région régulatrice de transcription d'un gène régulé par des androgènes et une partie à terminaison carboxy provenant du gène ERG.

7. Méthode selon la revendication 1, dans laquelle la fusion génique est une fusion d'un gène régulé par des androgènes et du gène ERG, et la méthode comprenant en outre l'étape consistant à caractériser les cellules de prostate en se basant sur la présence ou l'absence d'une délétion génomique dans la fusion génique.

8. Méthode selon la revendication 7, dans laquelle ladite délétion génomique est une délétion d'ADN génomique entre le gène TMPRSS2 et le gène ERG sur le chromosome 21.

9. Méthode selon la revendication 8, dans laquelle ladite délétion comprend la délétion de l'exon 1 du gène ERG, ou dans laquelle ladite délétion comprend la délétion de l'exon 3 du gène TMPRSS2.

10. Méthode selon la revendication 8, dans laquelle ladite délétion est une délétion comprise entre 2,8 et 2,85 mégabases d'ADN génomique.

11. Méthode selon la revendication 10, dans laquelle ladite délétion est détectée au moyen d'un essai FISH qui utilise au moins une sonde marquée par fluorescence choisie dans le groupe constitué par RP11-95121, CTD-2506J13, RP11-476D17 et RP11-24A11.

12. Méthode selon la revendication 10, dans laquelle la présence de la délétion indique un cancer de la prostate métastatique chez le sujet.

13. Méthode selon la revendication 1, comprenant en outre la coloration des cellules de prostate dudit échantillon par le biais d'une coloration à l'hématoxyline et l'éosine visant à déterminer la présence de cellules luminales atypiques ayant une taille nucléaire agrandie sans nucléoles visibles par rapport à des cellules adjacentes normales.

14. Méthode selon la revendication 1, comprenant en outre la détermination de la présence d'un antigène prostatique spécifique (APS) dans les cellules prostatiques dudit échantillon.

15. Méthode selon la revendication 1, dans laquelle ledit sujet est exempt du cancer de la prostate tel que déterminé par biopsie à l'aiguille.
